(19) Europäisches Patentamt
European Patent Office
Office européen des brevets

(11) Veröffentlichungsnummer: **0 036 155**
**A2**

(12) # EUROPÄISCHE PATENTANMELDUNG

(21) Anmeldenummer: 81101675.7

(22) Anmeldetag: 07.03.81

(51) Int. Cl.³: C 07 F 9/10
C 08 F 30/02, B 01 J 13/02
A 61 K 9/58

(30) Priorität: 17.03.80 DE 3010185

(43) Veröffentlichungstag der Anmeldung: 23.09.81 Patentblatt 81/38

(84) Benannte Vertragsstaaten: AT BE CH DE FR GB IT LI NL SE

(71) Anmelder: Max-Planck-Gesellschaft zur Förderung der Wissenschaften e.V.
Bunsenstrasse 10
D-3400 Göttingen(DE)

(72) Erfinder: Eibl, Hansjörg, Dr.
Steinweg 51
D-3406 Bovenden(DE)

(72) Erfinder: Nicksch, Alfar
Paul-Gerhardt-Ring 31
D-1000 Berlin 20(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) **Polymerisierbare Phospholipide, Verfahren zu ihrer Herstellung, polymere Phospholipide, Verfahren zu ihrer Herstellung sowie Verwendung der polymeren Phospholipide.**

(57) Es werden polymerisierbare Phospholipide der allgemeinen Formel I

$$(CH_2=\overset{R^1}{\overset{|}{C}}-\overset{O}{\overset{\|}{C}}-)_n-A \qquad (I)$$

in der

n eine ganze Zahl mit einem Wert von 1 bis 6,
$R^1$ ein Wasserstoffatom oder eine Methylgruppe und
A den Rest eines über den polaren oder den apolaren Bereich des Moleküls gebundenen Phospholipids oder Phospholipidanalogen der allgemeinen Formel II

$$R-PO_4H-Z \qquad (II)$$

in der
R für den lipophilen und
Z für den hydrophilen Anteil des Phospholipidmoleküls stehen,

bedeuten, und ein Verfahren zu ihrer Herstellung sowie aus jenen herstellbare polymere Phospholipide und deren Verwendung beschrieben.

EP 0 036 155 A2

Croydon Printing Company Ltd.

MAX-PLANCK-GESELLSCHAFT ZUR FÖRDERUNG DER WISSENSCHAFTEN E.V. HOE 80/S 011 Dr.HA

# Polymerisierbare Phospholipide, Verfahren zu ihrer Herstellung, polymere Phospholipide, Verfahren zu ihrer Herstellung sowie Verwendung der polymeren Phospholipide

Gegenstand der Erfindung sind polymerisierbare Phospholipide, ein Verfahren zu ihrer Herstellung, polymere Phospholipide, die aus diesen polymerisierbaren Phospholipiden gebildet werden können, ein Verfahren zu ihrer Herstellung und ihre Verwendung zur Herstellung von Mikrokapseln.

Es ist bereits bekannt, eine Vielzahl von natürlichen oder synthetischen Materialien für die Bildung von Mikrokapseln zu verwenden, die dazu dienen können, Arzneimittelwirkstoffe oder sonstige Wirksubstanzen, wie Enzyme, einzukapseln, zu stabilisieren und bis zu ihrer bestimmungsgemäßen Benutzung zu schützen.

Die zur Ausbildung von Mikrokapseln herkömmlich verwendeten Materialien sind beispielsweise synthetische Polymere, wie Polyamide, oder natürliche Materialien, wie Gelatine. Diese Materialien vermögen jedoch nicht vollständig zu befriedigen, da sie zum Teil für die Bildung der Mikrokapseln Mehr-Komponenten-Systeme unter Anwendung organischer Lösungsmittel benötigen, nicht die erforderliche Stabilität besitzen, biologisch nicht abgebaut werden können oder schädliche Nebenwirkungen entfalten können, wenn sie für das Verkapseln von Arzeimittelwirkstoffen eingesetzt werden sollen.

Die Aufgabe der vorliegenden Erfindung besteht nun darin, ein Material zu schaffen, das sich wesentlich besser als die herkömmlich eingesetzten Materialien dazu eignet, Wirksubstanzen einzukapseln oder anderweitig zu immobilisieren.

Es hat sich nun überraschenderweise gezeigt, daß man aus polymerisierbaren Phospholipiden polymere Phospholipide bilden kann, die aufgrund ihres Lipidcharakters befähigt sind, in wäßrigem Medium spontan organisierte Strukturen (Vesikel, synthetische Liposomen) in der Größe von ca. o,o5 bis 1oo /um auszubilden. Diese Teilchen stellen Hohlkugeln dar und sind daher für die Mikroverkapselung von wasserlöslichen Arzneimittelwirkstoffen oder anderen Wirkstoffen geeignet. Weiterhin lassen sich lipophile Substanzen in solchen Mikrokapseln anreichern, wenn sie, wie z. B. Cholesterin, im hydrophoben Teil der Polymeren löslich sind.

Gegenstand der Erfindung sind daher die polymerisierbaren Phospholipide nach Anspruch 1, ein Verfahren zu ihrer Herstellung nach Anspruch 6, die daraus gebildeten polymeren Phospholipide nach Anspruch 7, das Verfahren zu ihrer Herstellung gemäß Anspruch 9 und ihre Verwendung gemäß Anspruch 12. Die Unteransprüche betreffen besonders bevorzugte Ausführungsformen dieses Erfindungsgegenstandes.

Gegenstand der Erfindung sind insbesondere die polymerisierbaren Phospholipide der nachstehenden allgemeinen Formel I

$$(CH_2 = \overset{\overset{\displaystyle R^1}{|}}{C} - \overset{\overset{\displaystyle O}{\|}}{C} -)_n - A \qquad (I)$$

in der

n eine ganze Zahl mit einem Wert von 1 bis 6,

$R^1$ ein Wasserstoffatom oder eine Methylgruppe und

A den Rest eines über den polaren oder apolaren Bereich des Moleküls gebundenen Phospholipids oder Phospholipidanalogen der allgemeinen Formel II

$$R - PO_4H - Z \qquad (II)$$

in der R für den lipophilen und Z für den hydrophilen Anteil des Phospholipidmoleküls stehen, bedeuten.

Vorzugsweise steht der Rest A der obigen allgemeinen Formel I für den Rest eines aus natürlichem Material, wie Eigelb, Soja oder dergleichen, isolierten Phospholipids, das derivatisierbare funktionelle Gruppe, wie Hydroxylgruppen oder Aminogruppen enthält, wie z. B. für einen Kephalin-, Phosphatidylserin-, Phosphatidylglycerin-, Phosphatidylinositol-, Cardiolipin-, Spingomyelin- oder Cerebrosid-Rest.

Gemäß einer bevorzugten Ausführungsform der Erfindung entsprechen die polymerisierbaren Phospholipide der nachstehenden allgemeinen Formel III

$$R - PO_4H - Z - (- \overset{\overset{O}{\parallel}}{C} - \overset{\overset{R^1}{|}}{C} = CH_2)_n \qquad (III)$$

in der n eine ganze Zahl mit einem Wert von 1 bis 6 und $R^1$ ein Wasserstoffatom oder eine Methylgruppe bedeuten und der Rest R für einen Rest steht, der aus den nachstehend definierten Gruppen A) bis K) ausgewählt ist:

Die Gruppe A umfaßt Reste einwertiger Alkohole, wie von Hexanol, Dekanol, Hexadekanol, Eicosanol und Hexeicosanol sowie von Cyclohexanol und entsprechenden Verbindungen, die halogeniert sind, wie z. B. Bromhexanol-(1), 1o-Bromdekanol-(1) und 16-Bromhexadekanol-(1), oder ungesättigt sind, wie 9-Octadecenol-(1).

Die Gruppe B beinhaltet Reste von Verbindungen, die durch Monosubstitution von Alkandiolen unter Verätherung oder Veresterung entstanden sind, wie Acetylglykol, Äthylglykol, Dekanoylglykol, Decylglykol, Hexadekanoylglykol, Hexadecylglykol, Eicosanoylglykol, Eicosylglykol und entsprechende Monoacyl- oder Monoalkylderivate von Propandiol-(1,3), Propandiol-(1,2), Hexandiol-(1,6), Dekandiol-(1,1o) und Hexadekandiol-(1,16), die ebenso wie die Glykolderivate auf bekannte Weise leicht dargestellt werden können.

Die Gruppe C umfaßt Reste von Verbindungen, die ebenfalls auf Alkandiolen aufgebaut sind, die aber säurelabile Äthergruppierungen, wie beispielsweise den Trityl- oder Tetrahydropyranolrest enthalten, wie 1-Tritylglykol, 1-Tetrahydropyranylglykol, 1-Trityl-propandiol-(1,3), 1-Tetrahydropyranyl-hexandiol-(1,6), 1-Trityldekandiol-(1,1o), 1-Tetrahydropyranyl-dekandiol-(1,1o) sowie 1-Tritylhexadekanol-(1,16) und 1-Tetrahydropyranyl-hexadekanol-(1,16). Auch diese Verbindungen können leicht auf bekannte Weise dargestellt werden.

Die Gruppe D umfaßt ebenfalls Reste von Verbindungen, die auf Alkandiolen aufgebaut sind, die aber Äthergruppierungen enthalten, die durch katalytische Hydrogenolyse wieder entfernt werden können, beispielsweise Benzyläther, wie 1-Benzylglykol, 1-Benzyl-propandiol-(1,3), 1-Benzyl-propandiol-(1,2), 1-Benzylhexandiol-(1,6), 1-Benzyldekandiol-(1,1o), 1-Benzyl-hexadekandiol-(1,16). Auch diese Verbindungen können leicht auf bekannte Weise hergestellt werden.

Die Gruppe E umfaßt Reste von Verbindungen, die auf dem Glyceringrundgerüst aufgebaut sind und wobei zwei Alkoholfunktionen wie bei den Resten der Gruppe B sub-

stituiert sind, wie Reste von 1,2-Dimethylglycerin, 1,3-Dimethylglycerin, 1,2-Diacetylglycerin, 1,3-Diacetylglycerin und 1,2-Diäthylglycerin, 1,3-Diäthylglycerin sowie von entsprechenden Estern mit höheren Fettsäureresten, wie Caprinsäure, Laurinsäure, Palmitinsäure, Arachinsäure, Ölsäure und Linolsäure, oder von Äthern mit höheren Alkylresten, wie Decyl-, Hexadecyl-, Hedacosyl- sowie die gemischten Ester, Äther oder Ester-Äther-Verbindungen. Die entsprechenden Ausgangsprodukte sind nach bekannten Verfahren herstellbar.

Die Gruppe F umfaßt Reste von Glycerinderivaten, die säurelabile Gruppen, wie Ditritylreste, aufweisen, wie z. B. 1,3-Ditritylglycerin oder 1,2-Isopropylidenglycerin.

Die Gruppe G umfaßt Reste von Verbindungen, die auf Glycerin aufgebaut sind, die aber, wie bei den Resten der Gruppe D, Äthergruppierungen enthalten, die durch katalytische Hydrogenolyse wieder entfernt werden könne, beispielsweise Benzyläther, wie 1-Benzyl-2-lauroylglycerin, 1-Lauroyl-2-benzyl-glycerin, 1-Benzyl-2-palmitoyl-glycerin, 1-Palmitoyl-2-benzyl-glycerin, 1-Benzyl-2-arachoyl-glycerin, 1-Arachoyl-2-benzyl-glycerin sowie die entsprechenden Alkyläther.

Die Gruppe H umfaßt Reste von Verbindungen des Typs der Reste der Gruppen B und E, jedoch mit Polyalkoholen als Grundgerüst. Ein n-Polyol enthält dabei stets n-1 Substituenten und eine freie Hydroxylgruppe, die dann mit bekannten Verfahren zum Phosphorsäurediester ($R-PO_4H-Z$) umgesetzt werden kann. Beispiele für geeignete Polyalkohole sind Erythrit, Arabit, Xylit und Adonit, Mannit, Sorbit und Dulcit.

Die Gruppe I umfaßt Reste von Verbindungen des Typs der Reste der Gruppen C und F auf Basis von Polyalkoholen, die wieder säurelabil sind. Dabei sind in einem n-Polyol n-1 Gruppen geschützt, d. h. es ist eine freie primäre oder sekundäre Alkoholgruppe vorhanden, die wieder zur Umsetzung zum Phosphorsäurediester zur Verfügung steht.

Die Gruppe J umfaßt Reste von Verbindungen des Typs der Reste der Gruppen D und G, die Äthergruppierungen enthalten, die durch katalytische Hydrogenolyse wieder entfernt werden können.

Die Gruppe K umfaßt die Reste sonstiger biologisch und pharmazeutisch interessanter lipophiler Alkohole, die eine primäre oder sekundäre Hydroxylgruppe enthalten, wie z. B. von Cholesterin, Retinol, Androsteron, Ergosteron und anderen Steroidalkoholen, Isoprenoidalkoholen und Carotenoidalkoholen.

Die Gruppe Z der obigen allgemeinen Formel III steht für den polaren Molekülanteil, der über die Phosphatgruppe an den Rest R gebunden ist, und entspricht den nachstehend angegebenen Formeln 1) bis 6), wobei die in der Gruppierung rechts stehenden Heteroatome (O bzw. N) das Bindeglied zu dem polymerisierbaren Rest (Acrylsäurerest bzw. Methacrylsäurerest) darstellen.

| | |
|---|---|
| 1) $-(CH_2)_a-NH-$ | wobei a einen Wert von 2 bis 1o besitzt, |
| 2) $-(CH_2)_b-O-$ | wobei b einen Wert von 2 bis 1o besitzt, |

3) $-(CH_2)-(CHOH)_c-CH_2-O-$ wobei c einen Wert von 1 bis 4 besitzt,

4) $-(CH_2)_x-\overset{CH_3}{\underset{CH_3}{N^+}}-(CH_2)_y-O-$ wobei x und y unabhängig voneinander Werte von 2 bis 1o annehmen können,

5) $-CH_2-\overset{H}{\underset{COOH}{C}}-NH-$

oder

6) $-(CH_2)_x-\overset{CH_3}{\underset{CH_3}{N^+}}-(CH_2)_y-NH-$ wobei x und y unabhängig voneinander Werte von 2 bis 1o annehmen können.

Es können aber auch mehrere Hydroxylgruppen oder Aminogruppen im Molekülrest Z mit dem polymerisierbaren Rest verknüpft sein. Hierdurch kann man vernetzte Polymere bilden, die als Filtermaterialien, beispielsweise als Tabakfilter, eingesetzt werden können.

Gemäß einer weiteren Ausführungsform der Erfindung entsprechen die polymerisierbaren Phospholipide der nachstehenden alltemeinen Formel IV

$$(CH_2 = \overset{R^1}{C} - \overset{O}{\overset{\|}{C}} - )_n - R - PO_4H - Z \qquad (IV)$$

wobei in diesem Fall die polymerisierbare Gruppe im apolaren Bereich R des Moleküls gebunden ist. In dieser allgemeinen Formel IV besitzen die Symbole $R^1$ und n die oben angegebenen Bedeutungen, während R

A) für einen mindestens bifunktionellen Alkylrest mit 6 bis 22, bevorzugt 12 bis 22 Kohlenstoffatomen, wie z. B. einen Alkandiol-, Aminoalkohol- oder Alkandiaminrest;

B) für den Rest eines monosubstituierten Alkandiols, Aminoalkohols, Alkandiamins mit 2 bis 6 Kohlenstoffatomen, die einen Substituenten mit 6 bis 22, bevorzugt 12 bis 22 Kohlenstoffatomen mit Ester-, Äther- oder Amidverknüpfung aufweisen, der mindestens eine zusätzliche Amino- oder Alkoholfunktion enthält;

C) für den Rest eines mono- oder disubstituierten Glyverinderivats, das Substituenten wie unter B) genannt aufweist; oder

D) für den Rest eines mono- oder mehrfach substituierten ($n_{OH}$-1) Polyols, das Substituenten wie unter B) genannt aufweist,

steht, und

Z eine Gruppe der nachstehenden allgemeinen Formeln 1) bis 1o) oder ein Wasserstoffatom darstellt:

1) $-(CH_2)_a-NH_2$ wobei a einen Wert von 2 bis 1o besitzt,

2) $-(CH_2)_b-OH$ wobei b einen Wert von 2 bis 1o besitzt,

3) $-(CH_2)-(CHOH)_c-CH_2-OH$ wobei c einen Wert von 1 bis 4 besitzt,

4) $-(CH_2)_x-\overset{CH_3}{\underset{CH_3}{N^+}}-(CH_2)_y-OH$ wobei x und y unabhängig voneinander Werte von 2 bis 1o annehmen können.

5) $-CH_2-\overset{H}{\underset{COOH}{C}}-NH_2$

6) $-(CH_2)_x-\overset{CH_3}{\underset{CH_3}{N^+}}-(CH_2)_y-NH_2$ wobei x und y unabhängig voneinander Werte von 2 bis 1o annehmen können,

7) $-CH_2)_d-\overset{+}{N}CH_3H_2$ wobei d eine ganze Zahl von 2 bis 1o bedeutet,

8) $-(CH_2)_e-\overset{+}{N}(CH_3)_2H$ wobei e eine ganze Zahl von 2 bis 1o bedeutet,

9) $-(CH_2)_f-\overset{+}{N}(CH_3)_3$ wobei f eine ganze Zahl von 2 bis 1o bedeutet,

1o) $-(CH_2)_g-H$ wobei g eine ganze Zahl von 2 bis 1o bedeutet.

Die erfindungsgemäßen polymerisierbaren Phospholipide können dadurch hergestellt werden, daß man die polymerisierbaren Acrylsäure- bzw. Methacrylsäurereste über das jeweilige Säurechlorid oder mit Hilfe anderer gängiger Kondensationsmethoden unter Bildung der entsprechenden monomeren Ester oder Amide, in das Phospholipid oder das Phospholipidanaloge einführt.

Diese Verfahrensweise besteht darin, daß man ein reaktives Acrylsäurederivat der nachstehenden allgemeinen Formel V

$$CH_2 = \overset{R^1}{C} - \overset{O}{\overset{\|}{C}} - X \qquad (V)$$

in der X ein Halogenatom, eine Hydroxylgruppe oder eine andere leicht substituierbare Molekülgruppe darstellt und $R^1$ für ein Wasserstoffatom oder eine Methylgruppe

steht, in an sich bekannter Weise mit einem Phospholipid der nachstehenden allgemeinen Formel II

$$R - PO_4H - Z \qquad (II)$$

in der R und Z die oben angegebenen Bedeutungen besitzen, kondensiert.

Dabei kann die Kondensationsreaktion in der Weise geführt werden, daß die polymerisierbare funktionelle Gruppe einmal im polaren und einmal im apolaren Molekülbereich des Phospholipids bzw. der phospholipidanalogen Verbindung lokalisiert ist, wodurch sich die Verbindungen der oben angesprochenen allgemeinen Formeln III und IV ergeben.

Die erfindungsgemäßen polymerisierbaren Phospholipide können durch Homopolymerisation oder durch Copolymerisation unter Zuhilfenahme von üblichen Polymerisationsinitiatoren, wie α,α'-Azobisisobutyronitril, Dibenzoylperoxid, Riboflavin, einem Peroxydisulfat oder dergleichen, zu polymeren Phospholipiden der nachstehenden allgemeinen Formel VI

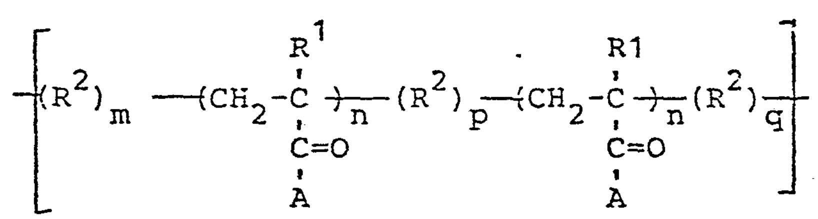
(VI)

polymerisiert werden, die ebenfalls Gegenstand der Erfindung sind. In der obigen allgemeinen Formel VI besitzen n, $R^1$ und A die oben angegebenen Bedeutungen, während $R^2$ für den Rest gleichartiger oder verschiedener copolymerisierbarer Monomerer steht und m, p und q unabhängig voneinander ganze Zahlen mit Werten von 0

bis 6 bedeuten. Vorzugsweise steht der Rest $R^2$ für den Rest einer copolymerisierbaren Acryl- oder Vinylverbindung, wie Acrylsäure, Methacrylsäure, Acrylsäure- oder Methacrylsäurealkylester mit jeweils 1 bis 22 Kohlenstoffatomen in der Alkylgruppe, Vinylchlorid, Vinylfluorid, Vinylbromid oder Vinylester oder Vinyläther mit jeweils 1 bis 22 Kohlenstoffatomen in der Alkylgruppe. Der Polymerisationsgrad dieser erfindungsgemäßen polymeren Phospholipide liegt oberhalb 1o, was einer unteren Grenze für das Molekulargewicht von ca. 8ooo entspricht.

Die Homo- oder Copolymerisation der erfindungsgemäßen polymerisierbaren Phospholipide unter Bildung der polymeren Phospholipide der oben angesprochenen allgemeinen Formel VI kann
a) lösungsmittelfrei (in der Masse),
b) in homogener Lösung (Lösungspolymerisation) oder
c) in wäßriger Emulsion (Emulsionspolymerisation)
unter Zuhilfenahme der oben angesprochenen Polymerisationsinitiatoren und unter Anwendung üblicher Verfahrensweisen durchgeführt werden. Vorzugsweise bewirkt man eine Blockmischpolymerisation.

Die erfindungsgemäßen polymeren Phospholipide der oben angesprochenen allgemeinen Formel VI sind aufgrund ihres Lipidcharakters befähigt, in wäßrigem Medium spontan organisierte Strukturen (Vesikel, synthetische Liposomen) in der Größe von ca. o,o5 bis 1oo /um auszubilden. Diese Partikel stellen Hohlkugeln dar und sind daher für die Mikroverkapselung von wasserlöslichen pharmazeutischen Wirkstoffen oder anderen Wirkstoffen geeignet. Lipophile Substanzen lassen sich ebenfalls in solchen Mikrokapseln anreichern, wenn sie, wie z. B. Cholesterin, in dem hydrophoben Teil der Polymeren löslich sind.

Gegenstand der Erfindung ist daher auch die Verwendung polymeren Phospholipide der oben angesprochenen allgemeinen Formel I zur Herstellung von Mikrokapseln.

Methodisch wird die Mikroverkapselung durch Polymerisation der erfindungsgemäßen polymerisierbaren Phospholipide der allgemeinen Formeln I bzw. III und IV in Gegenwart der zu verkapselnden Substanz erreicht. Zur Reinigung und Isolierung der beladenen Mikrokapseln haben sich Methoden, wie die Dialyse, die Zentrifugation und die Säulenchromatographie bewährt.

Der Vorteil der unter Einsatz der erfindungsgemäßen polymerisierbaren Phospholipide gebildeten Mikrokapseln aus dem polymeren Phospholipid der vorliegenden Erfindung gegenüber synthetischen Liposomen aus einfachen Lipiden liegt in der höheren Stabilität der erfindungsgemäßen polymeren Phospholipide.

Der Vorteil gegenüber Mikrokapseln aus herkömmlichen Polymermaterialien, wie Polyamiden, liegt

1) in der schonenderen Methode der Verkapselung, die hier in wäßrigem Medium erfolgt, so daß kein Zwei-Komponenten-System mit organischem Lösungsmittel notwendig ist, und
2) in der Tatsache, daß die erfindungsgemäßen polymeren Phospholipide den natürlich vorkommenden Membrankomponenten sehr ähnlich sind, d. h. es ist eine weitgehende biologische Abbaubarkeit gewährleistet und eine immunologische Indifferenz zu erwarten.

Da die Randbedingungen (Temperatur von weniger als 6o°C, wäßriges Medium) sehr schonend gestaltet werden können, ist erfindungsgemäß auch die Verkapselung sehr empfindlicher Substanzen, wie von Enzymen, möglich. Daraus ergibt sich die Möglichkeit der Immobilisierung von Enzymen ohne daß dieser über eine kovalente Bindung fixiert werden müssen.

Die erfindungsgemäßen polymeren Phospholipide stellen eine immobilisierte Lipidmatrix dar und können daher als Adsorbentien für lipophile Verbindungen dienen, beispielsweise als Trägermaterial für die Affinitätschromatographie oder als Stabilisator für lipophile Proteine.

Die erfindungsgemäßen polymeren Phospholipide können auch durch Polymerisation in Gegenwart von üblichen Vernetzern gebildet werden, wodurch man vernetzte Polymere erhält. Als Vernetzungsmittel kann man auch polymerisierbare Phospholipide der oben angegebenen allgemeinen Formel I einsetzen, die zwei zur Polymerisation geeignete ungesättigte Gruppen aufweisen.

Die folgenden Beispiele dienen der weiteren Erläuterung der Erfindung.

Dabei betreffen die Beispiele 1 bis 9 die Synthese polymerisierbarer Phospholipide, die im polaren Molekülbereich eine oder mehrere polymerisierbare funktionelle Gruppen aufweisen, die Beispiele 1o bis 12 die Synthese polymerisierbarer Phospholipide, die im apolaren Molekülbereich eine oder mehrere polymerisierbare funktionelle Gruppen aufweisen, die Beispiele 13 bis 18 die Herstellung der erfindungsgemäßen polymeren Phospholipide und das Beispiel 19 deren Verwendung zur Herstellung von Mikrokapseln.

B e i s p i e l 1

Die Acrylamidgruppen aufweisenden polymerisierbaren Phospholipide erhält man unter Anwendung der nachstehend angegebenen Verfahrensweise.

N-Methacrylamido-(1,2-dimyristoyl-rac-glycerin-3-phosphoryl)-äthanolamin

6,4 g ($1o^{-2}$ Mol) 1,2-Dimyristoyl-rac-glycerin-3-phosphoryl-äthanolamin in 1oo ml Trichloräthylen werden mit 2 g ($2 \times 1o^{-2}$ Mol) Triäthylamin versetzt. Unter Eiskühlung und Rühren wird eine Lösung von 2,1 g ($1,5 \times 1o^{-2}$ Mol) Methylacrylsäurechlorid in 5o ml Chloroform eingetropft. Nach dem Eintropfen wird das Reaktionsgemisch noch eine Stunde bei Raumtemperatur weitergerührt; danach werden das ausgefallene Triäthylammoniumhydrochlorid abgenutscht und das Filtrat am Rotationsverdampfer bei 25°C zur Trockne (Öl) eingeengt. Der Rückstand wird mit 15o ml gesättigter wäßriger Natriumchloridlösung behandelt und das Produkt mit Chloroform extrahiert. Der über Natriumsulfat getrocknete Chloroformextrakt wird auf 1o ml eingeengt und auf eine Kieselgelsäule (Merck HR 6o) aufgetragen. Fließmittelsystem $CHCl_3$ : $CH_3OH$ : $H_2O$ 1) 2oo : 15 : 1; 2) 1oo : 15 : 1; 3) 65 : 15 : 1 (V/V). Die säulenchromatographische Reinigung ergibt 5,5 g (78 % d. Th.) einer farblosen, bei Raumtemperatur gerade noch festen Substanz.

B e i s p i e l 2

Die Herstellung der Acrylestergruppen aufweisenden erfindungsgemäßen polymerisierbaren Phospholipide erfolgt unter Anwendung der nachstehenden Verfahrensweise:

Octadecyl-phosphorylglycerin-mono-methacrylsäureester

4,5 g ($10^{-2}$ Mol) Octadecyl-phosphorylglycerin werden in 5o ml Trichloräthylen gelöst, mit 2 g ($2 \times 10^{-2}$ Mol) Triäthylamin versetzt und unter Rühren und Eiskühlung während 2o Minuten zu einer Lösung von 2,1 g ($1,5 \times 10^{-2}$ Mol) Methacrylsäurechlorid in 5o ml Trichloräthylen getropft. Nach 2 Stunden wird das entstandene Triäthylammoniumhydrochlorid abgenutscht und das Filtrat am Rotationsverdampfer bei 3o°C im Wasserstrahlvakuum zur Trockne eingeengt. Zur Hydrolyse wird der Rückstand mit 2oo ml gesättiger Kochsalzlösung behandelt, wonach das Produkt mit Chloroform extrahiert wird. Die Chloroformphase wird über Natriumsulfat getrocknet und eingeengt, worauf das Produkt säulenchromatographisch gereinigt wird (Kieselgel Merck HR 6o; Fließmittelsystem $CHCl_3$ : $CH_3OH$ : $H_2O$ 1oo : 15 : 1 (V/V)). Man erhält 3,5 g (68 % d. Th.) eines bei Raumtemperatur hochviskosen, farblosen Öls.

Analyse: Octadecyl-phosphorylglycerin-mono-methacrylsäureester

$C_{25}H_{48}NaO_7P$; MG 514,6o

| | C % | H % | P % |
|---|---|---|---|
| berechnet: | 58,35 | 9,4o | 6,o2 |
| gefunden: | 58,29 | 9,3o | 5,79 |

B e i s p i e l 3

Octadecyl-phosphoryl-N-methacrylamino-äthanolamin

Man bildet diese Verbindung nach der Verfahrensweise von Beispiel 1 durch Umsetzen von Octadecylphosphoryläthanolamin mit Methacrylsäurechlorid. Die Verbindung

wird dünnschichtchromatographisch identifiziert.

Summenformel: $C_{24}H_{47}NNa\ O_5P$; MG: 483,54

B e i s p i e l 4

1,2-Dipentadecylmethyliden-glycerin-3-phosphoryl-N-acrylamido-äthanolamin

Man bildet die Titelverbindung durch Umsetzen von 1,2-Dipentadecylmethyliden-glycerin-3-phosphoryl-äthanolamin mit Acrylsäurechlorid nach der Verfahrensweise von Beispiel 1.

Summenformel: $C_{39}H_{75}NNaO_7P$; MG 723,97

| | C % | H % | N % | P % |
|---|---|---|---|---|
| berechnet: | 64,7o | 1o,44 | 1,93 | 4,28 |
| gefunden: | 65,1o | 1o,28 | 1,89 | 4,14 |

B e i s p i e l 5

N-Acrylamido-kephalin

Man bildet die Titelverbindung nach der Verfahrensweise von Beispiel 1 durch Umsetzung von isoliertem Ei-Kephalin und Acrylsäurechlorid. Die Verbindung wird dünnschichtchromatographisch identifiziert.

B e i s p i e l 6

Octadecylphosphoryl-propandiol-1,3-methacrylsäureester

Man bildet die Titelverbindung nach der Verfahrensweise von Beispiel 2 durch Umsetzen von Octadecylphosphorylpropandiol-1,3 mit Methacrylsäurechlorid.

Summenformel: $C_{25}H_{48}NaO_6P$, MG: 498,6o

| | C % | H % | P % |
|---|---|---|---|
| berechnet: | 6o,22 | 9,7o | 6,21 |
| gefunden: | 58,97 | 9,42 | 6,o2 |

B e i s p i e l 7

Palmitoylpropandiol-1,3-phosphoryl-N,N-dimethyl-N-{(ß-acryloyl)-äthyl}-äthanolamin

Man bildet die Titelverbindung nach der Verfahrensweise von Beispiel 2 und identifiziert die Verbindung dünnschichtchromatographisch.

Summenformel: $C_{28}H_{54}NO_8P$, MG: 563,69

B e i s p i e l 8

1,2-Dimyristoyl-sn-glycerin-3-phosphorylpropandiol-1,3-methacrylsäureester

Nach der Verfahrensweise von Beispiel 1 bildet man die Titelverbindung durch Umsetzen von 1,2-Dimyristoyl-sn-glycerin-3-phosphoryl-propandiol-1,3 mit Methacrylsäurechlorid. Man identifiziert die Verbindung dünnschichtchromatographisch.

Summenformel: $C_{38}H_{70}NaO_{10}P$, MG: 74o,92

B e i s p i e l 9

1,2-Dimyristoyl-sn-glycerin-3-phosphorylglycerin-monomethacrylsäureester

Man bildet die Titelverbindung nach der Verfahrensweise von Beispiel 2 durch Umsetzen von 1,2-Dimyristoyl-sn-glycerin-3-phosphoryl-glycerin mit Methacrylsäurechlorid.

Summenformel: $C_{38}H_{70}NaO_{11}P$, MG: 756,92

| | C % | H % | P % |
|---|---|---|---|
| berechnet: | 60,29 | 9,32 | 4,09 |
| gefunden: | 59,73 | 9,22 | 4,12 |

Bei den nachfolgenden Beispielen 10 bis 12 führt man den polymerisierbaren Rest in Analogie zu den in den obigen Beispielen angegebenen Verfahrensweisen nach Entfernung der Schutzgruppe (n) durch entsprechende Reaktionen (Hydrogenolyse, saure Spaltung) in die Phospholipidverbindung oder die phospholipidanaloge Verbindung ein.

B e i s p i e l 10

(16-O-Acryloyl)-hexadekanoyl-propandiol-1,3-phosphorylcholin

Man bildet diese Verbindung durch Umsetzung von 16-Hydroxyhexadekanoyl-propandiol-1,3-phosphorylcholin mit Acrylsäurechlorid.

Summenformel: $C_{27}H_{52}NO_8P \cdot H_2O$, MG: 567,69

| | C % | H % | N % | P % |
|---|---|---|---|---|
| berechnet: | 57,12 | 9,59 | 2,47 | 5,46 |
| gefunden: | 56,92 | 9,33 | 2,32 | 5,23 |

B e i s p i e l 11

1,2-Di-(16-O-acryloyl)-hexadekanoyl-rac-glycerin-3-phosphorylcholin

Man bildet die Titelverbindung durch Umsetzen von 1,2-Di-(16-hydroxy)-hexadekanoyl-rac-glycerin-3-phosphorylcholin mit Acrylsäurechlorid. Die Verbindung wird dünnschichtchromatographisch identifiziert.

Summenformel: $C_{44}H_{84}NO_{12}P \cdot H_2O$, MG: 868,11

B e i s p i e l 12

(12-Methacryloyl)-octadekanoyl-propandiol-1,3-phosphorylcholin

Man bildet die Titelverbindung durch Umsetzen von 12-Hydroxystearoylpropandiol-1,3-phosphorylcholin mit Methacrylsäurechlorid. Die Verbindung wird dünnschichtchromatographisch identifiziert.

Summenformel: $C_{30}H_{58}NO_8P \cdot H_2O$, MG: 609,76

Die nachstehenden Beispiele 13 bis 18 verdeutlichen die Herstellung der erfindungsgemäßen polymeren Phospholipide.

B e i s p i e l 13

Blockpolymerisation

Die allgemeine Verfahrensweise besteht darin, zu 1 g des geschmolzenen Monomeren (die Acryl- bzw. Methacrylverbindungen mit einer Alkylkette liegen bei Raumtemperatur als

hochviskose Öle vor, während die Verbindungen mit zwei langkettigen Alkylresten im Bereich zwischen 2o und 5o°C schmelzen) unter Rühren 1o bis 2o mg (1 bis 2 Gew.-%) Dibenzoylperoxid oder eine gleich große Menge α,α'-Azo-isobuttersäurenitril zuzugeben. Dieses Gemisch wird dann während 12 bis 24 Stunden bei 5o bis 6o°C in einem geschlossenen Reaktionsgefäß unter einer Stickstoffatmosphäre polymerisiert. Das feste Produkt wird zerkleinert und mehrfach mit einer Chloroform/Methanol-Mischung (1/1, V/V) ausgekocht, bis sich in der überstehenden Flüssigkeit dünnschichtchromatographisch kein Monomeres mehr nachweisen läßt. Die Ausbeuten betragen je nach Polymerisationstemperatur und Reaktionsdauer 5o bis 95 % der Theorie.

In der oben angegebenen allgemeinen Verfahrensweise bildet man bei einer Polymerisationstemperatur von 6o°C und einer Polymerisationsdauer von 3o Stunden unter Verwendung von Dibenzoylperoxid als Initiator Poly-(palmitoyl-2,2-dimethylpropandiol-(1,3)-phosphoryläthanolamino)-methacrylamid mit einer Ausbeute von 87 % der Theorie.

Summenformel: $(C_{27}H_{51}NNaP)_p$, MG: p·(555,65)

| | C % | H % | N % | P % |
|---|---|---|---|---|
| berechnet: | 58,25 | 9,42 | 2,52 | 5,56 |
| gefunden: | 58,46 | 8,75 | 2,2o | 4,75 |

B e i s p i e l 14

Lösungspolymerisation

Die Lösungspolymerisation wird allgemein in der Weise durchgeführt, daß man 1 g des Monomeren wahlweise entweder in 1o ml Dioxan oder in 1o ml Äthylacetat löst

und mit 1o mg (1 Gew.-%) Dibenzoylperoxid (alternativ $\alpha,\alpha'$-Diazoisobuttersäurenitril) versetzt. Dann erhitzt man die homogene Lösung während 24 Stunden im geschlossenen Gefäß unter einer Stickstoffatmosphäre auf 5o°C. Anschließend gießt man die erhaltene hochviskose Lösung in 15o ml Aceton, wobei das polymere Produkt ausfällt, das abgenutscht und im Vakuumexsiccator getrocknet wird. Die Ausbeuten liegen bei 7o bis 9o % der Theorie.

Unter Anwendung der oben beschriebenen Verfahrensweise bildet man Poly-(octadecyl-phosphorylglycerin)-methacrylsäureester.

Summenformel: $(C_{25}H_{49}O_7P)_p$, MG: p.(492,64)

| | C % | H % | P % |
|---|---|---|---|
| berechnet: | 6o,95 | 1o,o3 | 6,25 |
| gefunden: | 6o,o2 | 9,55 | 6,23 |

B e i s p i e l 15

Poly-(1,2-dipalmitoyl-rac-glycerin-3-phosphorylpropan-diol-(1,3))-methacrylsäureester

Man bildet die Titelverbindung nach der Verfahrensweise von Beispiel 14.

Summenformel: $(C_{42}H_{78}NaO_{10}P)_p$, MG: p.(797,o)

| | C % | H % | P % |
|---|---|---|---|
| berechnet: | 63,29 | 9,86 | 3,87 |
| gefunden: | 62,57 | 9,62 | 3,79 |

B e i s p i e l 16

Poly-(palmitoyl-2,2-dimethylpropandiol-(1,3)-phosphoryläthanolamino)-methacrylamid

Man bildet die Titelverbindung nach der Verfahrensweise von Beispiel 14.

Summenformel: $(C_{27}H_{51}NNaO_7P)_p$, MG: p.(555,65)

| | C % | H % | N % | P % |
|---|---|---|---|---|
| berechnet: | 58,25 | 9,42 | 2,52 | 5,56 |
| gefunden: | 57,41 | 9,o6 | 2,33 | 4,72 |
| | 58,88 | 9,95 | 4,17 | 5,61 |

B e i s p i e l 17

Poly-(1,2-dimyristoyl-sn-glycerin-3-phosphoryläthanolamino)-acrylamid

Man bildet die Titelverbindung nach der Verfahrensweise von Beispiel 14.

Summenformel: $(C_{36}H_{67}NNaO_9P)_p$, MG: p.(712,87)

| | C % | H % | N % | P % |
|---|---|---|---|---|
| berechnet: | 6o,65 | 9,62 | 1,96 | 4,35 |
| gefunden: | 59,99 | 9,25 | 2,78 | 4,o3 |

B e i s p i e l 18

Emulsionspolymerisation

A) Thermisch ausgelöste Emulsionspolymerisation
Man emulgiert 5 x $1o^{-4}$ Mol des Monomeren in 1oo ml

wäßriger Pufferlösung (Phosphatpuffer mit einem pH-Wert von 7,o) unter kräftigem Schütteln in einer Stickstoffatomosphäre. Danach versetzt man die Emulsion mit 1o bis 2o mg Kaliumperoxodisulfat, spült den Kolben erneut kräftig mit Stickstoff und erhitzt nach dem Verschließen für 12 bis 14 Stunden in einem Schüttelwasserbad auf eine Temperatur von 6o°C. Man verfolgt das Fortschreiten der Polymerisationsreaktion dünnschichtchromatographisch (Fließmittelsystem Chloroform/Methanol/25 %-iger wäßriger Ammoniak (65/15/1)). Man benutzt hierfür Chloroformextrakte von Proben der wäßrigen Reaktionsmischung. Der Umsatz wird aus den Dünnschichtchromatogrammen abgeschätzt.

B) Photochemisch ausgelöste Emulsionspolymerisation
Man emulgiert 5 x $1o^{-4}$ Mol des Monomeren in 1oo ml einer 5 x $1o^{-3}$ %-igen wäßrigen Lösung von Riboflavin-di-Na-Salz. Man temperiert den die Emulsion enthaltenden Kolben durch Umspülen mit Wasser. Dann bestrahlt man die Emulsion während 3o Minuten mit einer 1ooo W-Tageslichtlampe. Während dieser Zeit polymerisieren etwa 8o % des Monomeren. Die Umsatzkontrolle erfolgt dünnschichtchromatographisch. Bei dieser photochemisch ausgelösten Emulsionspolymerisation spielt die Wahl der Reaktionstemperatur eine wichtige Rolle.

Unter Anwendung der oben beschriebenen Verfahrensweisen und der in den Beispielen 14 bis 17 angegebenen Monomeren bildet man die entsprechenden Polymeren.

B e i s p i e l 19

Fluorescein enthaltende Mikrokapseln aus Poly-(octadecylphosphoryl-glycerin)-methacrylsäureester

Man löst 2,5 g (5 x $1o^{-3}$ Mol) Octadecylphosphorylglycerin-mono-methacrylsäureester in 1oo ml Chloroform und engt das Material bei Raumtemperatur im Wasserstrahlvakuum derart zur Trockne ein, daß die Innenseite des 1 l-Rundkolbens mit einer gleichmäßigen Schicht des Monomeren bedeckt ist. Dann beschickt man den Kolben mit 5oo ml einer o,1m Natriumchloridlösung, die 165 mg (5 x $1o^{-4}$ Mol) Fluorescein enthält und mit Natriumhydroxid auf einen pH-Wert von 7 bis 8 eingestellt worden ist. Nach Zugabe von 5o ml Kaliumperoxodisulfat als radikalischer Polymerisationsinitiator (der in einer Menge von 2 %, bezogen auf das Monomere, eingesetzt wird) und kräftigem Spülen mit Stickstoff verschließt man den Kolben dicht und erhitzt das Reaktionsgemisch während 4 Stunden im Schüttelwasserbad auf 6o°C. Dann überführt man 3o ml des erhaltenen Reaktionsgemisches zur Entfernung des nicht eingekapselten Fluoresceins in einen Dialysierschlauch aus regenerierter Cellulose (die für Substanzen mit einem Molekulargewicht von mehr als 15ooo permeabel ist) und dialysiert das Material während 48 Stunden gegen viermal 1 l einer o,1m Natriumchloridlösung. Die in dieser Weise erhaltenen Mikrokapseln sind mit Fluorescein gefüllt. Ihre Größe läßt sich fluoreszenzmikroskopisch und durch Elektronenmikroskopie bestimmen. Der Durchmesser dieser Mikrokapseln beträgt o,o5 bis 1o /um. Eine Auftrennung dieser Mikrokapseln in verschiedene Größenklassen ist chromatographisch möglich.

Aus dem obigen Beispiel ist ohne weiteres ersichtlich,

daß es mit Hilfe der erfindungsgemäßen Verfahrensweise gelingt, stabile Mikrokapseln unter sehr milden Bedingungen zu bilden, so daß auch empfindliche Substanzen, wie Enzyme, schonend verkapselt werden können, was mit Hilfe der bisher üblichen Bildung der Mikrokapseln durch Grenzflächenpolymerisation von Polyamiden nicht möglich war.

Patentansprüche:

1. Polymerisierbare Phospholipide der allgemeinen Formel I

$$\left(CH_2 = \overset{R^1}{\overset{|}{C}} - \overset{O}{\overset{\|}{C}} -\right)_n - A \qquad (I)$$

in der

n eine ganze Zahl mit einem Wert von 1 bis 6,

$R^1$ ein Wasserstoffatom oder eine Methylgruppe und

A den Rest eines über den polaren oder den apolaren Bereich des Moleküls gebundenen Phospholipids oder Phospholipidanalogen der allgemeinen Formel II

$$R - PO_4H - Z \qquad (II)$$

in der R für den lipophilen und Z für den hydrophilen Anteil des Phospholipidmoleküls stehen,

bedeuten.

2. Polymerisierbare Phospholipide nach Anspruch 1, dadurch gekennzeichnet, daß A für den Rest eines aus natürlichem Material isolierten Phospholipids steht, das derivatisierbare, funktionelle Gruppen (Alkohol oder Aminofunktionen) enthält, wie z. B. Kephalin, Phosphatidylserin, Phosphatidylglycerin, Phosphatidylinositol, Cardiolipin, Sphingomyelin, Cerebroside usw.

3. Polymerisierbare Phospholipide nach Anspruch 1, bei denen die polymerisierbare Gruppe im polaren Bereich Z des Phospholipidmoleküls gebunden ist, der allgemeinen Formel III

$$R - PO_4H - Z - (- \overset{O}{\overset{\|}{C}} - \overset{R^1}{\overset{|}{C}} = CH_2)_n \qquad (III)$$

in der

R

A) für den Rest eines einwertigen, gegebenenfalls halogenierten und/oder ungesättigten Alkohols;

B) für den Rest einer durch Monosubstitution von Alkandiolen unter Verätherung oder Veresterung gebildeten Verbindung;

C) für den Rest einer durch Monosubstitution von Alkandiolen unter Verätherung gebildeten, säurelabile Äthergruppen aufweisenden Verbindung;

D) für den Rest einer durch Monosubstitution von Alkandiolen unter Verätherung gebildeten, durch katalytische Hydrogenolyse abspaltbare Äthergruppen aufweisenden Verbindung;

E) für den Rest eines Glycerinderivats mit zwei verätherten und/oder veresterten Alkoholfunktionen;

F) für den Rest eines zwei säurelabile Äthergruppen aufweisenden Glycerinderivats;

G) für den Rest eines durch katalytische Hydrogenolyse abspaltbare Äthergruppen und gegebenenfalls Alkyläthergruppen aufweisenden Glycerinderivats;

H) für den Rest eines Phenols, dessen freie Hydroxylgruppen veräthert oder verestert sind;

I) für den Rest eines Polyols, dessen freie Hydroxylgruppen in säurelabile Äthergruppen umgewandelt sind;

J) für den Rest eines Polyols, dessen freie Hydroxylgruppen in durch katalytische Hydrogenolyse abspaltbare Äthergruppen umgewandelt sind; oder

K) für den Rest eines lipophilen Alkohols

steht und

Z eine Gruppe der allgemeinen Formeln

1) $-(CH_2)_a-NH-$ wobei a einen Wert von 2 bis 1o besitzt,

2) $-(CH_2)_b-O-$ wobei b einen Wert von 2 bis 1o besitzt,

3) $-(CH_2)-(CHOH)_c-CH_2-O-$ wobei c einen Wert von 1 bis 4 besitzt,

4) $-(CH_2)_x-\overset{CH_3}{\underset{CH_3}{N^+}}-(CH_2)_y-O-$ wobei x und y unabhängig voneinander Werte von 2 bis 1o annehmen können,

5) $-CH_2-\overset{H}{\underset{COOH}{C}}-NH-$

oder

6) $-(CH_2)_x-\overset{CH_3}{\underset{CH_3}{N^+}}-(CH_2)_y-NH-$ wobei x und y unabhängig voneinander Werte von 2 bis 1o annehmen können,

n eine ganze Zahl mit einem Wert von 1 bis 6 und
$R^1$ ein Wasserstoffatom oder eine Methylgruppe
bedeuten.

4. Polymerisierbare Phospholipide nach Anspruch 3, dadurch gekennzeichnet, daß
R

A) für den Rest von Hexanol, Dekanol, Hexadekanol, Eicosanol, Hexaeicosanol, Cyclohexanol, Bromhexanol-(1), 1o-Bromdekanol-(1), 16-Bromhexadekanol-(1) oder 9-Octadecenol-(1);

B) für den Rest von Acetylglykol, Äthylglykol, Dekanoylglykol, Decylglykol, Hexadekanoylglykol, Hexadecylglykol, Eicosanoylglykol, Eicosylglykol oder der Monoacyl- oder Monoalkylderivate von Propandiol-(1,3), Propandiol-(1,2), Hexandiol-(1,6), Dekandiol-(1,1o) und Hexadekandiol-(1,16), wobei die Acyl- und Alkylgruppen der Monoacyl- oder Monoalkylderivate jeweils 1 bis 22 Kohlenstoffatome aufweisen;

C) für den Rest von 1-Tritylglykol, 1-Tetrahydropyranylglykol, 1-Trityl-propandiol-(1,3), 1-Tetrahydropyranyl-propandiol-(1,3), 1-Tritylhexandiol-(1,6), 1-Tetrahydropyranyl-hexandiol-(1,6), 1-Trityl-dekandiol-(1,1o), 1-Tetrahydropyranyl-dekandiol-(1,1o), 1-Tritylhexadekanol-(1,16) oder 1-Tetrahydropyranyl-hexadekanol-(1,16);

D) für den Rest von 1-Benzylglykol, 1-Benzyl-propandiol-(1,3), 1-Benzyl-propandiol-(1,2), 1-Benzylhexandiol-(1,6), 1-Benzyldekandiol-(1,1o) oder 1-Benzyl-hexadekandiol-(1,16);

E) für den Rest von 1,2-Dimethylglycerin, 1,3-Dimethylglycerin, 1,2-Diacetylglycerin, 1,3-Diacetylglycerin, 1,2-Diäthylglycerin, 1,3-Diäthylglycerin oder der entsprechenden Ester mit höheren Fettsäuren, wie Caprinsäure, Laurinsäure, Palmitinsäure, Arachinsäure, Ölsäure oder Linolsäure oder der entsprechenden Äther mit höheren Alkylresten, wie Dekanresten, Hexadecylresten oder Hexacosylresten, sowie der entsprechenden gemischten Ester, gemischten Äther oder gemischten Ester-Äther-Verbindungen;

F) für den Rest von 1,3-Ditritylglycerin oder 1,2-Isopropylidenglycerin;

G) für den Rest von 1-Benzyl-2-lauroyl-glycerin, 1-Lauroyl-2-benzyl-glycerin, 1-Benzyl-2-palmitoyl-glycerin, 1-Palmitoyl-2-benzyl-glycerin, 1-Benzyl-2-arachoyl-glycerin oder 1-Arachoyl-2-benzyl-glycerin oder der entsprechenden Alkyläther mit niedrigmolekularen Alkylgruppen;

H) für den Rest von Erythrit, Arabit, Xylit, Adonit, Mannit, Sorbit oder Dulcit; oder

K) für den Rest von Cholesterin, Retinol, Androsteron, Ergosteron, eines Steroidalkohols, eines Isoprenoidalkohols oder eines Carotenoidalkohols

steht.

5. Polymerisierbare Phospholipide nach Anspruch 1, bei denen die polymerisierbare Gruppe im apolaren Bereich R des Moleküls gebunden ist, der allgemeinen Formel IV

$$(CH_2 = \overset{R^1}{\overset{|}{C}} - \overset{O}{\overset{\|}{C}} - )_n - R - PO_4H - Z \qquad (IV)$$

in der

R

A) für einen mindestens bifunktionellen Alkylrest mit

6 bis 22, bevorzugt 12 bis 22 Kohlenstoffatomen, wie z.B. einen Alkandiol-, Aminoalkohol- oder Alkandiaminrest;

B) für den Rest eines monosubstituierten Alkandiols, Aminoalkohols, Alkandiamins mit 2 bis 6 Kohlenstoffatomen, die einen Substituenten mit 6 bis 22, bevorzugt 12 bis 22 Kohlenstoffatomen mit Ester-, Äther- oder Amidverknüpfung aufweisen, der mindestens eine zusätzliche Amino- oder Alkoholfunktion enthält;

C) für den Rest eines mono- oder disubstituierten Glycerinderivats, das Substituenten wie unter B) genannt aufweist; oder

D) für den Rest eines mono- oder mehrfach substituierten $(n_{OH}-1)$ Polyols, das Substituenten wie unter B) genannt aufweist,

steht und

Z eine Gruppe der allgemeinen Formeln:

1) $-(CH_2)_a-NH_2$ wobei a einen Wert von 2 bis 1o besitzt,

2) $-(CH_2)_b-OH$ wobei b einen Wert von 2 bis 1o besitzt,

3) $-(CH_2)-(CHOH)_c-CH_2-OH$ wobei c einen Wert von 1 bis 4 besitzt,

4) $-(CH_2)_x-\overset{CH_3}{\underset{CH_3}{\overset{|}{\underset{|}{N^+}}}}-(CH_2)_y-OH$ wobei x und y unabhängig voneinander Werte von 2 bis 1o annehmen können,

5) $-CH_2-\overset{H}{\underset{COOH}{\overset{|}{\underset{|}{C}}}}-NH_2$

6) $-(CH_2)_x-\overset{CH_3}{\underset{CH_3}{\overset{|}{\underset{|}{N^+}}}}-(CH_2)_y-NH_2$ wobei x un y unabhängig voneinander Werte von 2 bis 1o annehmen können,

7) $-(CH_2)_d-\overset{+}{N}CH_3H_2$ wobei d eine ganze Zahl von 2 bis 1o bedeutet,

8) $-(CH_2)_e-\overset{+}{N}(CH_3)_2H$ wobei e eine ganze Zahl von 2 bis 1o bedeutet,

9) $-(CH_2)_f-\overset{+}{N}(CH_3)_3$ wobei f eine ganze Zahl von 2 bis 1o bedeutet,

1o) $-(CH_2)_g-H$ wobei g eine ganze Zahl von 2 bis 1o bedeutet,

oder

11) ein Wasserstoffatom,

n eine ganze Zahl mit einem Wert von 1 bis 6 und

$R^1$ ein Wasserstoffatom oder eine Methylgruppe

bedeuten.

6. Verfahren zur Herstellung der polymerisierbaren Phospholipide nach den Ansprüchen 1 bis 5, dadurch g e k e n n z e i c h n e t , daß man ein reaktives Acrylsäurederivat der allgemeinen Formel V

$$CH_2 = \overset{R^1}{C} - \overset{O}{\overset{\|}{C}} - X \qquad (V)$$

worin X ein Halogenatom, eine Hydroxylgruppe oder eine andere leicht substituierbare Molekülgruppe darstellt und $R^1$ für ein Wasserstoffatom oder eine Methylgruppe steht, in an sich bekannter Weise mit einem Phospholipid der allgemeinen Formel II

$$R - PO_4H - Z \qquad (II)$$

in der R und Z die in den Ansprüchen 1 bis 5 angegebenen Bedeutungen besitzen, kondensiert.

7. Polymere Phospholipide, dadurch g e k e n n z e i c h n e t , daß sie in ihrer Molekülkette wiederkehrende Einheiten der allgemeinen Formel VI

$$\left[ -(R^2)_m - \left( CH_2 - \underset{\underset{A}{|}}{\underset{C=O}{\overset{R^1}{\overset{|}{C}}}} \right)_n - (R^2)_p - \left( CH_2 - \underset{\underset{A}{|}}{\underset{C=O}{\overset{R^1}{\overset{|}{C}}}} \right)_n - (R^2)_q - \right] \qquad (VI)$$

enthalten, in der
n, $R^1$ und A die in den Ansprüchen 1 bis 5 angegebenen Bedeutungen besitzen,
$R^2$ für den Rest gleichartiger oder verschiedener copolymerisierbarer Monomeren steht und
m, p und q unabhängig voneinander ganze Zahlen mit Werten von o bis 6 bedeuten.

8. Polymere Phospholipide nach Anspruch 7, dadurch g e k e n n z e i c h n e t , daß $R^2$ für den Rest einer copolymerisierbaren Acryl- oder Vinyl-Verbindung, wie Acrylsäure, Methacrylsäure, Acrylsäure- und Methacrylsäurealkylester mit jeweils 1 bis 22 Kohlenstoffatomen in der Alkylgruppe, Vinylchlorid, Vinylfluorid, Vinylbromid, Vinylester oder Vinyläther mit jeweils 1 bis 22 Kohlenstoffatomen in der Alkylgruppe, steht.

9. Verfahren zur Herstellung der polymeren Phospholipide nach den Ansprüchen 7 und 8, dadurch g e k e n n z e i c h n e t , daß man eine Verbindung der allgemeinen Formel I

$$\left( CH_2 = \overset{R^1}{\overset{|}{C}} - \overset{O}{\overset{\|}{C}} - \right)_n - A \qquad (I)$$

worin $R^1$, n und A die in den Ansprüchen 1 bis 5 angegebenen Bedeutungen besitzen,
gegebenenfalls in Gegenwart eines copolymerisierbaren Monomeren in an sich bekannter Weise polymerisiert.

10. Verfahren nach Anspruch 9, dadurch gekennzeichnet, daß man eine Blockmischpolymerisation durchführt.

11. Verfahren nach den Ansprüchen 9 oder 10, dadurch gekennzeichnet, daß man die Polymerisation in der Masse, in Lösung oder als Emulsionspolymerisation durchführt.

12. Verwendung der polymeren Phospholipide nach den Ansprüchen 7 und 8 zur Herstellung von Mikrokapseln.

Patentansprüche für Österreich

1. Verfahren zur Herstellung polymerisierbarer Phospholipide der allgemeinen Formel I

$$\left( CH_2 = \overset{\overset{\displaystyle R^1}{|}}{C} - \overset{\overset{\displaystyle O}{\|}}{C} - \right)_n - A \qquad (I)$$

in der

n eine ganze Zahl mit einem Wert von 1 bis 6,

$R^1$ ein Wasserstoffatom oder eine Methylgruppe und

A den Rest eines über den polaren oder an apolaren Bereich des Moleküls gebundenen Phospholipids oder Phospholipidanalogen der allgemeinen Formel II

$$R - PO_4H - Z \qquad (II)$$

in der

P für den lipophilen und

Z für den hydrophilen Anteil des Phospholipidmoleküls stehen,

bedeuten, dadurch gekennzeichnet, daß man ein reaktives Acrylsäurederivat der allgemeinen Formel V

$$CH_2 = \overset{\overset{\displaystyle R^1}{|}}{C} - \overset{\overset{\displaystyle O}{\|}}{C} - X \qquad (V)$$

worin X ein Halogenatom, eine Hydroxylgruppe oder eine andere leicht substituierbare Molekülgruppe darstellt und

$R^1$ für ein Wasserstoffatom oder eine Methylgruppe

steht, in an sich bekannter Weise mit einem Phospholipid der allgemeinen Formel II kondensiert.

2. Verfahren zur Herstellung polymerer Phospholipide, die in ihrer Molekülkette wiederkehrende Einheiten der allgemeinen Formel VI

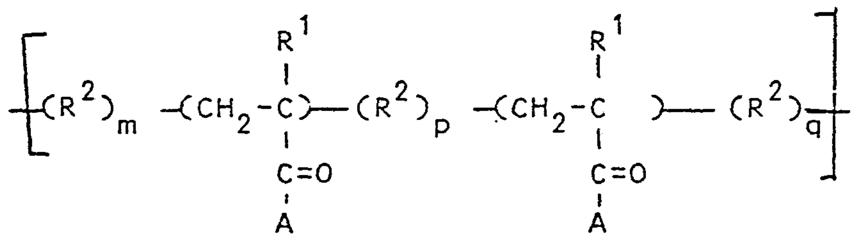

(VI)

enthalten, in der
n, $R^1$ und A die im Anspruch 1 angegebene Bedeutung besitzen,
$R^2$ für den Rest gleichartiger oder verschiedener copolymerisierbarer Monomeren steht und
m, p und q unabhängig voneinander ganze Zahlen mit Werten von 0 bis 6 bedeuten, dadurch gekennzeichnet, daß man eine Verbindung der allgemeinen Formel I aus Anspruch 1, worin $R^1$, n und A die im Anspruch 1 angegebene Bedeutung besitzen, gegebenenfalls in Gegenwart eines copolymerisierbaren Monomeren in an sich bekannter Weise polymerisiert.